# EUROPÄISCHE PATENTSCHRIFT

(11) **EP 1 157 265 B1**
(45) Veröffentlichungstag und Bekanntmachung des Hinweises auf die Patenterteilung: **27.08.2003**
(21) Anmeldenummer: 00945701.1
(22) Anmeldetag: 02.06.2000
(51) Int. Cl.: G01N 7/10

(54) **VERFAHREN ZUR MESSUNG DER KONZENTRATION ODER DES PARTIALDRUCKES VON GASEN, INSBESONDERE SAUERSTOFF, IN FLUIDEN UND GASSENSOR**
METHOD FOR MEASURING THE CONCENTRATION OR THE PARTIAL PRESSURE OF GASES, ESPECIALLY OXYGEN, IN FLUIDS AND A CORRESPONDING GAS SENSOR
PROCEDE POUR MESURER LA CONCENTRATION OU LA PRESSION PARTIELLE DE GAZ, NOTAMMENT D'OXYGENE, DANS DES FLUIDES ET CAPTEUR DE GAZ CORRESPONDANT

(30) Priorität: 01.06.1999 DE 19925842
(43) Veröffentlichungstag der Anmeldung: 28.11.2001
(73) Patentinhaber: UFZ-UMWELTFORSCHUNGSZENTRUM Leipzig-Halle GmbH, 04318 Leipzig (DE)
(72) Erfinder: LAZIK, Detlef, D-06198 Salzmünde (DE); GEISTLINGER, Helmut, Dr., D-04289 Leipzig (DE)
(74) Vertreter: Hengelhaupt, Jürgen D., Dipl.-Ing.
(86) Internationale Anmeldenummer: EP0005081
(87) Internationale Veröffentlichungsnummer: WO01053796

(56) Entgegenhaltungen:
- US-A- 3 871 228
- US-A- 4 112 737
- US-A- 4 662 210
- US-A- 5 763 762

## Beschreibung

Die Erfindung betrifft ein Verfahren zur Messung der Konzentration oder des Partialdruckes von Gasen, insbesondere Sauerstoff, in Fluiden sowie einen Gassensor zu dessen Durchführung.

Die Messung der Konzentration von Sauerstoff oder anderen Gasen ist auf verschiedenen technischen Gebieten, so in der chemischen Verfahrenstechnik, der Umwelttechnologie, der Medizintechnik usw. von hoher Wichtigkeit.

Traditionell wird gelöster Sauerstoff in elektrochemischen Messzellen nach dem Clark-Prinzip galvanisch, polarographisch oder potentiometrisch gemessen, das heißt, es wird der Strom als Funktion der Zeit oder des Potentials oder das Potential im stromlosen Zustand in einem Elektrolyten zwischen einer Kathode und einer Anode gemessen.

Um Sauerstoff in das Messzelleninnere zu führen, ist eine sauerstoff- bzw. gasdurchlässige Membran vorhanden, die den Elektrolyten in der Messzelle von dem zu messenden Fluid trennt.

Die Messung setzt die Gleichgewichtseinstellung der Partialdrücke außerhalb und innerhalb der Zelle, bzw. der damit über die Henrykonstante korrespondierenden Konzentrationen voraus. Da die elektrochemische Reaktion Sauerstoff zehrt, muss durch Rühren der Messzelle im Fluid eine konstante Anströmung erzeugt werden. Viele Hersteller bieten deshalb aufsteckbare Rührwerke an. Bekannt sind weitere Verfahren, die den Sauerstoffverbrauch kompensieren und deshalb nur für die Messung geringer Konzentrationen oder/und in kleinen Volumina geeignet sind. Die Messwerte müssen druckkompensiert, salinitäts- und temperaturkorrigiert werden.

Die Messung ist lokal am Ort des Sensors definiert. Probleme entstehen leicht durch Verunreinigungen an der relativ kleinen Membran und durch Lufteinschlüsse beim Membranwechsel.

Andere Möglichkeiten der Sauerstoffmessung bestehen beispielsweise in der Auswertung der sauerstoffkonzentrationsabhängigen Modulation der Phase eingestrahlten Lichtes.

Für die Konzentrationsmessung anderer Gase sind gasspezifische Methoden bekannt, sofern nicht auf die Gaschromatographie zurückgegriffen wird.

Weitere Meßverfahren sind aus den Schriften US-A 5, 763,762 (D1) und US-A 4,112,737 (D2) bekannt.

D1 zeigt ein Gerät zur Bestimmung des Gleichgewichtsdruckes der in einem Lösungsmittel vorhandenen Gase. Bei der angewandten Messmethode wird ein Gefäß (besser: eine Membran) am Messpunkt fixiert, worauf man wartet bis die Gaszusammensetzung in dem Gefäß gleich der der Umgebung ist. Ist Gleichgewichtsdruck erreicht, wird der Innendruck im Gefäß gemessen. In D1 ist als semipermeable Membran eine Kapillarröhre mit hoher Gasdurchlässigkeit gewählt, so dass ein Gleichgewicht in weniger als 5 Minuten hergestellt werden soll, siehe Sp. 3, Z. 54-56. Dem kommt ein besonderes Verhältnis von Membranoberfläche zu Gefäßinhalt entgegen.

D2 beschreibt ein Verfahren und ein Gerät, mit denen ein Gas (fault gas) über eine semipermeable Membran von einem Lösungsmittel (oil) getrennt wird und - nachdem Gleichgewichtsdruck (equilibrium) erreicht ist - einer analytischen Prozedur unterzogen wird (Gaschromatographie). Der Innendruck im Aufnahmegefäß wird hierbei nicht gemessen.

Alle genannten Verfahren erfordern eine komplizierte und empfindliche Sensorik, entsprechende Messgeräte sind deshalb kostenaufwendig und haben eine nur geringe Standzeit bzw. erfordern einen hohen Wartungs- und Kalibrieraufwand. Für Messungen unter rauhen Bedingungen, z. B. zur Messung des Sauerstoffpartialdrucks in Bodenschichten, sind sie gänzlich ungeeignet.

In tieferen Bodenschichten, z. B. im Grundwasser, ist die Gasmessung bisher auch deshalb nicht möglich, da eine Messung nur durch Probenentnahme erfolgen könnte. Da das Wasser aber unter entsprechend hohem Druck und spezieller Temperatur steht, würde es bei einer Entnahme und der Messung unter atmosphärischen Bedingungen entgasen und es würden keine dem Ort der Messung zuordenbaren Messwerte erzielt werden. Zudem erscheint die lokale Messung deshalb problematisch, da nicht eingeschätzt werden kann, welche Störung das System durch die Probenentnahme erfährt.

Der Erfindung liegt die Aufgabe zugrunde, ein Verfahren und einen geeigneten Gassensor der eingangs genannten Art anzugeben, die unkompliziert und mit einfachen Mitteln arbeiten, robust sind und zu repräsentativen Ergebnissen für die untersuchte Struktur führen.

Die Aufgabe wird erfindungsgemäß gelöst durch die Merkmale im kennzeichnenden Teil der Ansprüche 1 und 6 im Zusammenwirken mit den Merkmalen im Oberbegriff. Zweckmäßige Ausgestaltungen der Erfindung sind in den Unteransprüchen enthalten.

Überraschend wurde gefunden, dass sich die Eigenschaften sauerstoffdurchlässiger Kunststoffe wie z. B. PTFE oder anderer, gasspezifisch durchlässiger Kunststoffe dazu nutzen lassen, einen Sauerstoff- bzw. Gassensor aufzubauen, der es erlaubt, den Partialdruck oder die Konzentration des Sauerstoffs oder anderer Gase eines Gasgemisches durch eine direkte Druckmessung zu bestimmen, da der zu messende Partialdruck innerhalb eines beliebigen Fluids einen äquivalenten Druck in einem geschlossenen Gefäß hervorruft, dessen Wandung mindestens teilweise aus einem solchen sauerstoff- bzwgasdurchlässigen Kunststoff besteht.

PTFE (Polytetrafluorethylen) und PTFE-ähnliche Produkte, z. B. Teflon, ein Mischpolymerisat von PTFE und <5 Mol-% PAVE (Perfluoralkylvinylether), besitzen eine relativ hohe Durchlässigkeit für Sauerstoff. Gleichzeitig nimmt PTFE unter den Kunststoffen eine herausragende Bedeutung aufgrund seiner enormen Beständigkeit gegenüber beliebigen Einflüssen aus seiner Umgebung ein. Adhäsion und Kohäsion des Materials sind sehr gering, entsprechend klein werden damit auch Reibungskräfte. Die Oberflächen bleiben sauber.

PTFE besitzt eine weitere bemerkenswerte Eigenschaft. Die Durchlässigkeit des Kunststoffes z. B. gegenüber dem gut wasserlöslichen Kohlendioxid und dem in der Atmosphäre hauptsächlich vorkommenden Stickstoff liegt um zwei Größenordnungen unter der anderer Kunststoffe wie z. B. HDPE (high density polyethylene).

Die Nutzung der Eigenschaften
- Relativ hohe Sauerstoffdurchlässigkeit und
- vernachlässigbare Durchlässigkeit gegenüber anderen Flüssigkeiten und Gasen
führt mit dem erfindungsgemäß aufgebauten Gassensor dazu, dass der Partialdruck des Sauerstoffs in einem Stoffgemisch einen äquivalenten Partialdruck in einem geschlossenen Teflongefäß hervorruft und dass dieser, unabhängig von dem im Gefäß vorhandenen Absolutdruck und nur abhängig von dem zuvor im Gefäß vorhandenen Sauerstoffpartialdruck eine Änderung des absoluten Drucks hervorruft. Das heißt, die Messung der Sauerstoffkonzentration kann auf eine einfache Druckmessung reduziert werden.

Das Verfahren ist in beliebigen fluiden Medien anwendbar.

Der Druck wird z. B. mittels langzeit- und überlaststabilen, aber auch fast beliebig klein dimensionierbaren und in weitem Bereich linearen Messfühlern auf der Basis von einkristallinem Silizium mit einer Genauigkeit von etwa 0,4% gemessen. Damit kann von der Möglichkeit einer preiswerten und präzisen Sauerstoffpartialdruckmessung ausgegangen werden, wobei die Konzentration je nach Rand- und Anfangsbedingungen der Messung sowohl aus der Anstiegsgeschwindigkeit des Drucks bei bekannten Materialeigenschaften des verwendeten Kunststoffs und der Geometrie der Messzelle oder - ohne diese Kenntnisse - als Funktion des Absolutdruckunterschiedes ermittelt wird.

Eine weitere Variante besteht in der Messung mittels Differenzdruckgebern gegen ein Referenzgefäß, das bekannten Bedingungen, z. B. atmosphärischen Bedingungen unterliegt und das ansonsten möglichst gleich dem Messsystem konfiguriert ist, also insbesondere hinsichtlich der Materialauswahl des Messgefäßes sowie dessen Abmessungen.

Da durch die Wandung des Gefäßes auch Wasserdampf diffundiert sowie weitere Gase, wenn auch mit größeren Zeitkonstanten, wird vor einer erneuten Messung eine Kalibrierung vorgenommen, indem das Gefäß mit Stickstoff oder einem Gasgemisch bekannter Zusammensetzung bzw. Luft gespült wird. Durch Temperieren des Spülgases kann, sofern nötig, gleichzeitig eine bestimmte Messtemperatur in dem Gefäß eingestellt werden.

Der Messzellenaufbau und das Messprinzip können an das jeweilige Messproblem angepasst werden. Im einfachsten Fall besteht der Gassensor aus einem Hohlkörper, dessen eine Wandung mindestens teilweise durch eine Sauerstoff durchlässige Membran gebildet ist, und in dem ein Druckaufnehmer angeordnet ist. Neben dieser punktförmigen Messung (z. B. auch in kugelförmiger Geometrie der Messzelle) ist mit Hilfe einer schlauchförmigen Messanordnung jederzeit auch die integrierende Messung entlang einer Linie oder mit Hilfe eines Schlauchnetzwerkes die integrierende Messung einer Fläche möglich. Damit wird ein wesentliches Ziel innerhalb von Monitoringaufgaben in der Umwelt - die Ermittlung von Sauerstoff in der Bodenluft oder im Grundwasser heterogener Systeme - einfach und repräsentativ erreicht.

In-Situ-Gas-Monitoring ist bisher im allgemeinen nur punktförmig und qualitativ möglich. Wie bereits oben festgestellt, ist die am Messfühler registrierte isocore Druckänderung Resultante lokal begrenzter Diffusionsvorgänge, wenn der Druckausgleich in einem Messschlauch schnell gegenüber den lokal unterschiedlichen Ausgleichsvorgängen des Partialdruckes ist. Die Druckmessung in einem solchen Messschlauch integriert somit über alle lokal begrenzten Partialdruckänderungen, ohne dass der zu messende Stoff in unmittelbarem Kontakt mit dem Messfühler, nämlich dem Drucksensor, steht. Wenn an einer beliebigen Kontaktstelle des Außenraums mit der Sammelleitung ein Konzentrationsgradient für Sauerstoff zum Gasgemisch existiert, dann führt dieser infolge der Sauerstoff-Diffusion zur Erhöhung des Sauerstoffpartialdrucks im Schlauchinnern. Diese Partialdruckerhöhung an der Probeentnahmestelle wird an der Messstelle als Gesamtdruckerhöhung gemessen. Der erhöhte Sauerstoffpartialdruck muss nicht an der Messstelle, d. h. am Drucksensor selbst, vorliegen. Es muss also nicht eine gewisse Quantität von Sauerstoffmolekülen am Ort des Messfühlers vorhanden sein, da der Druck und die Druckänderung intensive Zustandsgrößen sind, die sich mit Schallgeschwindigkeit in dem Gasgemisch im Inneren eines Schlauches ausbreiten, und die damit unabhängig vom Ort innerhalb eines geschlossenen Systems sind. Die zeitliche Auflösung des Messsystems ist deshalb nur durch die relativ langsame Diffusionskinetik in die Messleitung bestimmt.

Für die In-Situ-Messung kann das Messgefäß, z. B. eine schlauchförmige Messleitung, fest im Untersuchungsobjekt verlegt werden und bedarf dort keiner Wartung.

Eine Möglichkeit zur räumlichen Begrenzung des oder der Bezugspunkte für die Messung besteht darin, die Sammlerleitung nur in bestimmten Bereichen gasselektiv auszugestalten. Die übrige Leitung kann dann z. B. aus einer Edelstahlkapillare bestehen.

Für Gase im geschlossenen System und unter atmosphärischem Druck gilt in guter Näherung die Zustandsgleichung des idealen Gases p*V/T = const. Bei konstanter Temperatur kann statt einer isocoren Druckänderung demnach auch die isobare Volumenänderung oder das Produkt von Druck- und Volumenänderung zur Messung herangezogen werden. Beide Größen liefern unmittelbar die Gasmengenänderung als Funktion der Zeit, wobei die Messung unter isobaren Bedingungen mathematisch einfacher zu interpretieren ist. Eine zusätzliche gasdichte Leitung kann der Ermittlung der mittleren Temperatur dienen.

Bringt man geeignete Sammelleitungen oder Netzwerke, gegebenenfalls höhendifferenziert, in Deponien, Bioreaktoren u. ä. ein, um z. B. die Sauerstoffverteilung beim aeroben Abbau von organischen Schadstoffen zu messen, dann ist das integrale Messprinzip nahezu ideal für diesen Anwendungszweck geeignet.

Ein Schlauchbündel mit unterschiedlichen Materialien liefert ein differenziertes Zeitverhalten von Druckoder Volumenänderungen, wobei die wesentliche und erste Änderung stets durch das selektiv bevorzugte Gas entsteht, untergeordnet sich jedoch die Diffusion der anderen Gasse im Verhältnis der Diffusionskonstanten auswirken werden. Es ist eine Vielzahl solcher Materialien mit unterschiedlicher Gasselektivität bekannt. Für eine Schlauchzahl des Bündels, die gleich der Zahl der erwarteten bzw. zu messenden Gase, gegebenenfalls plus einen zusätzlichen Schlauch ist, führt ein einfach lösbares, bestimmtes Gleichungssystem zur Ermittlung vom repräsentativen Partialdruck der Gase und der Temperatur in der Umgebung des Schlauchbündels, das als Materialparameter die Diffusionskonstanten Dᵢⱼ und die Temperaturleitfähigkeit λ_{j,} die Geometrie der Schläuche (Länge Lⱼ, Durchmesser 2Rⱼ, Wandstärke bⱼ der Schläuche) und die Messgrößen Druck pⱼ(t) oder Volumen Vⱼ(t) enthält (Index i für Gase, Index j für Schlauch).

Eine weitere Anwendungsmöglichkeit sind gebietsrepräsentative Gastracerversuche zur Ermittlung der Gasmigration im Untergrund des Bodens über parallel verlegte Schlauchbündel für wassergesättigte und ungesättigte Verhältnisse. Dabei kann beispielsweise durch die Kombination inerter und reaktiver Gase der Gasverbrauch infolge mikrobiellen Umsatzes oder anderer gaszehrender Prozesse in Summe ermittelt werden und steht als zuverlässige Größe zur thermodynamischen Interpretation von Reaktionen im Untergrund zur Verfügung.

Das Messprinzip eignet sich zur Bestimmung der Sauerstoffkonzentration, ggf. auch weiterer Gase, auch dort, wo bisher eine solche Messung auf große Probleme stieß, also beispielsweise die direkte Messung im Boden in größeren Tiefen oder die direkte Messung in Abwässern und Fließgewässern.

Die Erfindung soll nachstehend anhand eines Ausführungsbeispiels näher erläutert werden. In den zugehörigen Zeichnungen zeigen
- Fig. 1: eine schematische Darstellung eines erfindungsgemäßen Gassensors,
- Fig. 2: den Druckverlauf in dem Gefäß des Gassensors in einem Experiment und
- Fig. 3: den Druckverlauf in einem weiteren Experiment.

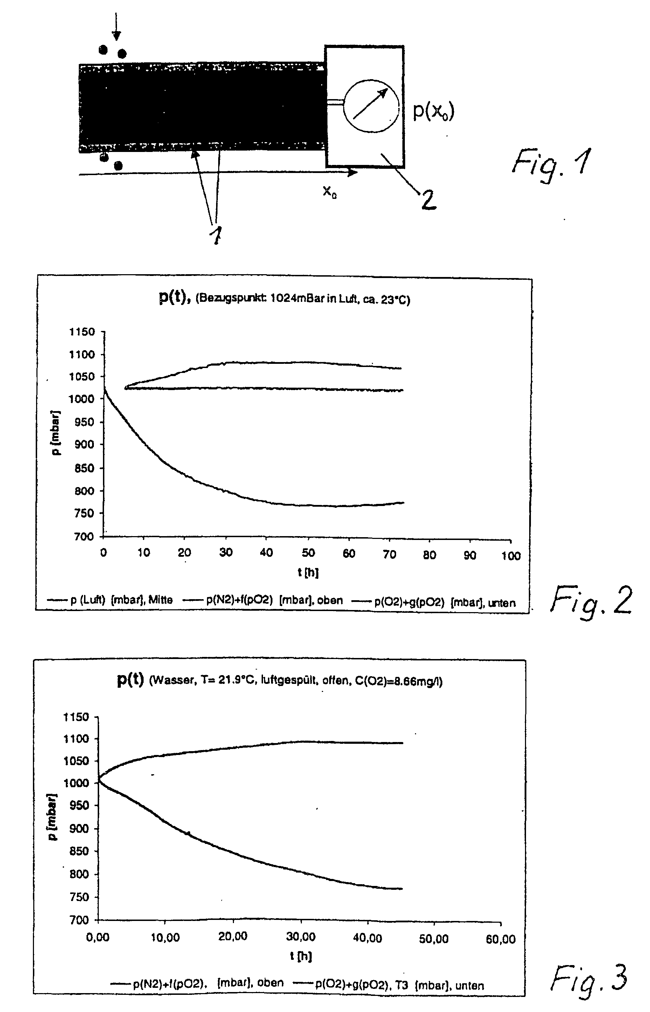

Fig. 1 zeigt einen Gassensor zur Ermittlung der Sauerstoffkonzentration in einem Fluid. Er besteht aus einem Schlauch mit einer Schlauchwandung 1 aus PTFE, der im vorliegenden Fall einseitig geschlossen ist. Er könnte andernfalls auch als Ring aufgebaut sein. Er vermag über seine gesamte Länge x Sauerstoff aus einem Fluid diffundieren zu lassen, beispielsweise zur Messung der Sauerstoffkonzentration im Wasser innerhalb einer Bodenschicht. Am Schlauchende befindet sich ein Drucksensor 2.

Der mit dem Drucksensor 2 gemessene Absolutdruck oder der Anstieg der Druckänderung sind ein Maß für den Sauerstoffpartialdruck in dem zu messenden Fluid.

Zum Funktionsnachweis des so aufgebauten Gassensors wurden vier Experimente durchgeführt, deren Ergebnis in den Fig. 2 und 3 dargestellt ist.

Ein mit Stickstoff gefüllter Teflonschlauch wurde bei einem Ausgangsinnen- und -außendruck von P = 1024 mbar wahlweise luftgespültem Wasser und Luft ausgesetzt. Am Schlauchende wurde ein Druckaufnehmer installiert. Der Absolutdruck wurde als Zeitfunktion aufgezeichnet. Der außerhalb des Schlauches vorhandene Sauerstoffpartialdruck rief in beiden Fällen einen Druckanstieg im Teflonschlauch hervor, der deutlich über den Umgebungsdruck stieg (obere Kurven in Fig. 2 und 3).

Ein mit Sauerstoff unter einem Druck von p = 1024 mbar gefüllter Teflonschlauch wurde wahlweise luftgespültem Wasser und Luft ausgesetzt. Am Schlauchende wurde wiederum ein Druckaufnehmer installiert und der Druck als Zeitfunktion aufgezeichnet. Der innerhalb des Schlauches vorhandene, gegenüber der Umgebung erhöhte Sauerstoffpartialdruck rief in diesen beiden Fällen einen Druckabfall im Teflonschlauch hervor, d. h. der Schlauchinnendruck fiel deutlich unter den Umgebungsdruck entsprechend der vom Partialdruckgefälle abhängigen Diffusion der Sauerstoffmoleküle nach außen (untere Kurven in Fig. 2 und 3).

Verwendet wurden, da es sich um reine Demonstrationsversuche handelte, relativ dickwandige Teflonschläuche mit einem Innendurchmesser >5mm. Infolgedessen weisen die ermittelten Druckfunktionen p = p(t) relativ hohe Zeitkonstanten auf. Für reale Messaufgaben lassen sich mit dünnwandigen Schläuchen bei kleinerem Volumen/Oberflächen-Verhältnis entsprechend geringere Zeitkonstanten erreichen. Andererseits kann die Wandungsstärke des Schlauches auch bewusst zur Unterdrückung kurzwelliger Fluktuationen des Partialdruckes von Sauerstoff gewählt werden.

### Bezugszeichenliste

- 1: Schlauchwandung
- 2: Drucksensor
- x: Länge

## Patentansprüche

1. Verfahren zur Messung der Konzentration oder des Partialdruckes von Gasen, insbesondere Sauerstoff, in Fluiden
**Dadurch gekennzeichnet, dass**
Im Inneren eines hohlen, nach außen abgeschlossenen Gefäßes, dessen Wandung mindestens teilweise aus einem gasspezifisch durchlässigen Kunststoffmaterial besteht, das mit seiner Außenseite mit dem Fluid in Berührung steht, die der Gaskonzentration proportionale Partialdruckänderung als Druckänderung oder als Änderung einer druckabhängigen physikalischen Größe gemessen wird, nachdem das Gefäß zuvor mit einem Gas oder Gasgemisch bekannter Zusammensetzung gespült wurde.

2. Verfahren nach Anspruch 1.
**gekennzeichnet dadurch, dass**
das Gefäß vor einer Messung mit Stickstoff gespült wird.

3. Verfahren nach Anspruch 1 oder 2,
**gekennzeichnet dadurch, dass**
das Gas oder Gasgemisch entsprechend den Messbedingungen temperiert wird.

4. Verfahren nach einem der vorhergehenden Ansprüche, **gekennzeichnet dadurch, dass**
die Druckmessung in mehreren mit dem Fluid in Berührung stehenden Gefäßen mit unterschiedlicher Gaspermeabilität vorgenommen wird und das Zeitverhalten der Messwerte zur Ermittlung der Konzentration mehrerer Gase im Fluid in einem Gleichungssystem zueinander in Beziehung gesetzt wird.

5. Verfahren nach einem der vorhergehenden Ansprüche, **gekennzeichnet dadurch, dass**
der jeweils in dem Gefäß gemessene Druck zu einem gleichartigen Referenzsystem in Beziehung gesetzt wird, das bekannten Bedingungen unterliegt.

6. Gassensor zur Messung der Konzentration oder des Partialdruckes von Gasen, insbesondere Sauerstoff, in Fluiden zur Durchführung des Verfahrens nach einem der Ansprüche 1 bis 5,
**gekennzeichnet durch**
mindestens ein hohles, nach außen abgeschlossenes Gefäß, das mindestens teilweise aus einem sauerstoff- oder gasspezifisch durchlässigen Kunststoffmaterial besteht, einem den Innendruck in dem Gefäß messenden Drucksensor und einem den Zeitverlauf des Innendruckes registrierenden Aufzetchnungsgerät.

7. Gassensor nach Anspruch 6,
**gekennzeichnet dadurch, dass**
das Kunststoffmaterial in Form einer das Gefäß dicht abschließenden Membran vorliegt.

8. Gassensor nach Anspruch 6,
**gekennzeichnet dadurch, dass**
das Kunststoffmaterial in Form eines mit den übrigen Teilen des Gefäßes dicht verbundenen Schlauches vorliegt.

9. Gassensor nach Anspruch 6,
**gekennzeichnet dadurch, dass**
das Kunststoffmaterial in Form eines mit den übrigen Teilen des Gefäßes dicht verbundenen Schlauchnetzwerkes vorliegt.

10. Gassensor nach Anspruch 6 bis 9,
**gekennzeichnet dadurch, dass**
das Kunststoffmaterial Polytetrafluorethylen ist.

## Claims

1. Method for the measurement of the concentration *or* the partial pressure of gases, particularly oxygen, in fluids
**characterized in that**
in the internal area of a hollow vessel closed off to the outside, whose wall is at least partially made of a gas-specific permeable synthetic material, the exterior of which is in contact with the fluid, the partial pressure change proportional to the gas concentration is measured as a time-scanning of pressure change or as a change of a pressure-dependent physical variable, when the vessel has been purged beforehand with a gas or a gas mixture having a known composition.

2. Method according to Claim 1,
**characterized in that**
the vessel, before a measurement, is purged with nitrogen.

3. Method according to Claim 1 or 2,
**characterized in that**
the gas or gas mixture is tempered in accordance with the measuring conditions.

4. Method according to one of the previous Claims
**characterized in that**
the pressure measurement is carried out in several vessels which are in contact with the fluid and which have varying gas permeability, and the timing behaviour of the measuring values for determining the concentration of several gases in the fluid is set in relationship to one another in an equation system.

5. Method according to one of the previous Claims
**characterized in that**
the pressure measured in each case in the vessel is set in relationship to a similar reference system, which is subject to known conditions.

6. Gas sensor for the measurement of the concentration or the partial pressure of gases, particularly oxygen, in fluids for the execution of the method according to one of the Claims 1 to 5,
**characterized in that**
there is at least one hollow vessel, closed off to the outside, consisting at least partially of an oxygen or gas-specific permeable synthetic material, and a pressure sensor measuring the internal pressure in the vessel, and a recording instrument which records the variation in time of the internal pressure.

7. Gas sensor according to Claim 6,
**characterized in that**
the synthetic material is presented in the form of a membrane, which tightly seals off the vessel.

8. Gas sensor according to Claim 6
**characterized in that**
the synthetic material is presented in the form of a hose tightly bound to the remaining parts of the vessel.

9. Gas sensor according to Claim 6,
**characterized in that**
the synthetic material is presented in the form of a hose network tightly bound to the remaining parts of the vessel.

10. Gas sensor according to Claims 6 to 9,
**characterized in that**
the synthetic material is polytetrafluor ethylene.

## Revendications

1. Procédé pour mesurer la concentration ou la pression partielle de gaz, notamment d'oxygène, dans des fluides,
**caractérisé en ce que**
à l'intérieur d'un récipient creux, fermé vers l'extérieur, dont la paroi se compose au moins partiellement d'un matériau en plastique perméable de manière spécifique aux gaz, qui est en contact avec son côté extérieur avec le fluide, la variation de pression partielle proportionnelle à la concentration de gaz est mesurée en tant que variation de pression ou en tant que variation d'une valeur physique dépendant de la pression, après que le récipient a au préalable été rincé avec un gaz ou un mélange de gaz de composition connue.

2. Procédé selon la revendication 1,
**caractérisé en ce que**
le récipient est rincé avec de l'azote avant une mesure.

3. Procédé selon la revendication 1 ou 2,
**caractérisé en ce que**
le gaz ou le mélange de gaz est équilibré en température en fonction des conditions de mesure.

4. Procédé selon l'une quelconque des revendications précédentes,
**caractérisé en ce que**
la mesure de la pression est effectuée dans plusieurs récipients en contact avec le fluide, de perméabilité aux gaz différente, et le comportement en fonction du temps des valeurs de mesure est rapporté dans un système d'équations pour déterminer la concentration de plusieurs gaz dans le fluide.

5. Procédé selon l'une quelconque des revendications précédentes,
**caractérisé en ce que**
la pression mesurée à chaque fois dans un récipient est rapportée à un système de référence similaire, qui est surbordonné à des conditions connues.

6. Capteur de gaz pour la mesure de la concentration ou de la pression partielle de gaz, notamment d'oxygène, dans des fluides, pour la mise en oeuvre du procédé selon l'une quelconque des revendications 1 à 5,
**caractérisé par**
au moins un récipient creux, fermé vers l'extérieur, qui se compose au moins partiellement d'un matériau en plastique perméable de manière spécifique à l'oxygène ou aux gaz,
un capteur de pression mesurant la pression à l'intérieur du récipient
et un appareil d'enregistrement enregistrant l'allure dans le temps de la pression interne.

7. Capteur de gaz selon la revendication 6,
**caractérisé en ce que**
le matériau en plastique se présente sous la forme d'une membrane fermant le récipient de manière hermétique.

8. Capteur de gaz selon la revendication 6,
**caractérisé en ce que**
le matériau en plastique se présente sous la forme d'un tuyau souple connecté hermétiquement aux autres parties du récipient.

9. Capteur de gaz selon la revendication 6,
**caractérisé en ce que**
le matériau en plastique se présente sous la forme d'un réseau de tuyaux souples connectés hermétiquement aux autres parties du récipient.

10. Capteur de gaz selon les revendications 6 à 9,
**caractérisé en ce que**
le matériau en plastique est du polytétrafluoroéthylène.
